# EUROPEAN PATENT SPECIFICATION

(11) **EP 0 532 716 B1**
(45) Date of publication and mention of the grant of the patent: **07.05.1997**
(21) Application number: 92902251.5
(22) Date of filing: 18.12.1991
(51) Int. Cl.: C07K 7/08, C07K 14/52, C07K 2/00, A61K 38/00, A61K 39/00, G01N 33/569

(54) **NEW SYNTHETHIC PEPTIDES WITH IMUNOMODULATING ACTIVITY AND PREPARATION THEREOF**
NEUE SYNTHETISCHE PEPTIDE MIT IMMUNOMODULATORISCHER WIRKUNG UND DEREN HERSTELLUNG
NOUVEAUX PEPTIDES SYNTHETIQUES AYANT UNE ACTIVITE IMUNOMODULATRICE ET LEUR PREPARATION

(30) Priority: 27.12.1990 GB 9028097
(43) Date of publication of application: 24.03.1993
(73) Proprietor: INSTITUTO CIENTIFICO Y TECNOLOGICO DE NAVARRA, S.A., 31008 Pamplona (ES)
(72) Inventor: SANTIAGO, Calvo Esteban, E-31080 Pamplona (ES); SUBIRA BADOS, Maria Luisa, E-31080 Pamplona (ES); BRUGAROLAS MASLLORENS, Antonio, E-31080 Pamplona (ES); LOPEZ MORATALLA, Natalia, E-31080 Pamplona (ES); LOPEZ ZABALZA, Maria Jesus, E-31080 Pamplona (ES); MARTIN ALGARRA, Salvador, E-31080 Pamplona (ES); BORRAS CUESTA, Francisco, E-31080 Pamplona (ES)
(74) Representative: Elzaburu Marquez, Alberto
(86) International application number: ES9100087
(87) International publication number: WO9212171

(56) References cited:
- Chemical Abstracts, volumen 114, numero 7, 18 Febrero 1991 (Columbus, Ohio, US) J. Golvano et al.: "Polarity of immunogens: implications for vaccine design", ver página 508, resumen 60086q, & Euro. J. Immunol 1990, 20(10), 2363-66
- Chemical Abstracts, volumen 108, num. 23, 6 Junio 1988 (Columbus, Ohio, US) F. Borras-Cuesta et al.: "Immunogenicity of synthetic peptides corresponding to regions of the major inner capsid protein of bovine rotavirus (BRV), ver página
- 507, resumen 202814c, & Ann. Inst. Pasteur/Virol 1987, 138(4), 437-50
- Chemical Abstracts, volumen 107, num. 21, 23 Noviembre 1987 (Columbus, Ohio, US) F. Boras-Cuesta et al.: "Engineering of immunogenic peptides by co-linear synthesis of determinants recognized by B and T cells", ver página 567, resumen 196073w, & Eur. J. Immunol. 1987, 17(8), 1213-15
- Chemical Abstracts, volumen 116, num. 4, 17 Febrero 1992 (Columbus, Ohio, US) J.G. Guillet et al.: "Haplotype specific homology scanning algorithm to predict T-cell epitopes from protein sequences", ver página 621, resumen 56809b, & J. Mol. Recognit. 1991, 4(1), 17-25

## Description

This invention relates to a series of new peptides active as modulators of the immune system. These peptides are useful in adoptive immunotherapy and as reagents in the assessment of cell mediated immunity. These new molecules, through their capacity to induce cytotoxic activity, are suitable for therapeutic use in diseases due to, or accompanied by, alterations in the immune system as in some types of cancer, infections and congenital or degenerative diseases. They can also be useful in the prophylaxis of immune dysfunctions.

### Background of the invention.

Immunologic mechanisms are known to be involved in the pathogenesis and development of certain conditions such as cancer, infections, and congenital or degenerative diseases. It is also known that the modulation of the immunologic alterations through the administration of molecules mediating cytotoxicity can change the natural evolution of these diseases, delaying the appearance of complications or even leading to their cure (Rosenberg SA, Mule JJ, Spies PJ, Reichert CM, Schwartz SL, J Exp Med (1985) 161:1169-1188). Proteins and peptides have been reported to participate in some of the mechanisms regulating the immune response. Recent work has led to important discoveries in this field leading to the identification of molecules involved in the normal or abnormal function of the immune system (Young RA, Elliott TJ, Cell (1989) 59:5-8; Marx J, Science (1990) 249:246-248); it has also led to the synthesis of new molecules with the aim of potentiating, modulating, or modifying the effects obtained with the natural products. The "in vitro" generation of cells with antitumour activity from lymphomononuclear cells present in peripheral blood, or from lymphomononuclear cells extracted from resected tumours, has shown that it is possible to modulate "ex vivo" the immune system in such a way that they are opening the door to new therapeutic approaches in diseases until now resistant to conventional treatments (Rosenberg SA. In De Vita VT, Hellman S, Rosenberg SA eds. Important advances in oncology. Philadelphia: JB Lippincott, 1986:55-91; Topalian SL, Rosenberg SA eds. Important advances in oncology. Philadelphia: JB Lippincott, 1990:19-41).

Proteins and peptides, as well as other compounds derived from amino acids or other chemical structures are already in use as immunomodulators in clinical practice. Proteins or peptides are the Interferon-gamma (The Merck Index 11 ed. N^{º} 4894), Interleukin-2 (The Merck Index 11 ed. N^{º} 4896), Thymomodulin (The Merck Index 11 ed. N^{º} 9338). Among the immunomodulators derived from amino acids a few compounds may be mentioned: Bucillamine (The Merck Index 11 ed. N^{º} 1447) y Ubenimex (The Merck Index 11 ed. Nº 9750). Another group of immunomodulators is related to mono- or polysaccharides. Representatives of this group are Amiprilose (The Merck Index 11 ed. Nº 499) y Lentinan (The Merck Index 11 ed. N^{º} 5322).

Other immunoestimulators possess a heterocyclic or mixed structure. Muroctasin (The Merck Index 11 ed. Nº 6217), Platonin (The Merck Index 11 ed. N^{º} 7504), Procodazole (The Merck Index 11 ed. N^{º} 7769) and Tetramisole (The Merck Index 11 ed. N^{º} 9161) belong to this group.

However, this type of treatment is still surrounded by controversy due to the severity of some of the side effects (Osband ME, Ross S, Immunology Today (1990) 13:193--195). For this reason this therapy is reserved to specialised centres where the immunologic factors to be considered for a correct management can be adequately assessed.

### DETAILED DESCRIPTION OF THE INVENTION

Based on the data existing in the literature regarding peptides that especifically bind to receptors involved in the stimulation of the immune system (Bjorkman PJ, Saper MA, Samraoni B, Bennett WS, Strominger JL, Wiley DC, Nature (1987) 329:506; White J, Herman A, Pullen AM, Kubo R, Kappler JW, Marrack P. Cell (1989) 56:27; Born W, Hall L, Dallas A, Boymel J, Schinnick T, Young D, Brennan P, O'Brien R, Science (1990) 249:67; Sette A, Buus S, Colon S, Smith JA, Miles C, Pierce SK, Proc Natl Acad Sci USA (1987) 84:1659), we have carried out the synthesis of the peptides, object of the present invention. These peptides induce cytotoxic activity mediated by lymphomononuclear cells. The peptides, object of the present invention, possess the general formula

R₁-aa₁-aa₂-aa₃-aa₄-aa₅-Pro-aa₇-aa₈-aa₉-aa₁₀-aa₁₁-aa₁₂-aa₁₃-aa₁₄-aa₁₅-R₂

in which
R₁ represents H, R₂ represents OH, or else R₁ and R₂ represent each an amino acid residue or peptide chains, equal or different, containing from 2 to 10 amino acid residues, in such a way that when R₁=H and R₂=OH, the formula represents a pentadecapeptide in which the N-terminal and the C-terminal'amino acids are respectively aa₁ and aa₁₅;
aa₂ is a Val, Leu, Ile, Ala, Lys or Gly residue;
aa₁₁ is a Glu or Asp residue;
and the rest of the amino acid residues aaₙ different from aa₂ and aa₁₁ may be any of the 20 natural amino acids with L configuration.

Therefore, the peptides of the invention may have from 15 amino acids (this is the case when R₁=H and R₂=OH) to 35 amino acids, when the 15 amino acid peptide is extended on both ends, thus R₁ and R₂ representing each from none up to 10 amino acids residues. The preferred structure is that in which R₁=H and R₂=OH, and readily soluble in water.

It must be clear that the general features of the peptides object of the present invention are defined by:
i) the position of Pro (between aa₅ and aa₇) with respect to the residues aa₂ and aa₁₁, each representing the aforementioned stated amino acid residues.
ii) the length of the peptide chains represented by R₁ and R₂, which may be equal of different; therefore the peptides object of the invention may have from 15 to 35 amino acids.

The chemical synthesis of these peptides has been carried our by the solid phase method of Merrifield (Merrifield RB; J Am Chem Soc (1963), 85:2149) with the Fmoc modification (Atherton E, Logan JC, Sheppard CR, J Chem Soc Perkin Trans (1981), 1:538).

According to this technique the synthesis of the peptides is carried out as follows:

### 1. Preparation of the resin:

The sarcosine polyamide gel resin is treated with diethylendiamine for 10 hours, and washed with enough dimethylformamide (DMF). The resin is then let to react with a linkage agent, preferably the pentafluorophenyl ester of the 4-hydroxymethylphenoxyacetic acid. Once the linkage agent has been incorporated, the Kaiser ninhydrine test is performed to ascertain the absence of free amino groups, and therefore should be negative.

### 2. Attachment of the first amino acid to the linkage arm of the resin:

The symmetric anhydride of the first amino acid with the amino group protected by the fluorene-methyloxycarbonyl group (Fmoc) is prepared. Any other groups present in the lateral chain of the amino acid should be equally protected with groups compatible with the Fmoc modification of the technique. The symmetric anhydride is let to react with the resin for 80 to 100 minutes using dimethylaminopyridine as catalytic agent. The resin is then washed with abundant dimethylformamide (DMF), and the Fmoc group is removed with 20% piperidine is DMF. The amino group is now ready to react with the carboxyl group of a new amino acid.

### 3. The peptide-forming step:

The rest of the amino acids are incorporated sequentially using their symmetric anhydrides or their active esters. The symmetric anhydrides are prepared from the corresponding amino acids activating them with dicyclohexylcarbodiimide in dichloromethane. The insoluble by-product dicyclohexylurea is removed by filtration; dichloromethane is eliminated in a rotating flash evaporator and the symmetric anhydride present in the residue is dissolved in DMF and added to the resin. The reaction is allowed to proceed for 60-90 minutes and the ninhydrin test is negative showing the absence of free amino groups (Kaiser E, Colescott RL, Bossinger CD, Cook PI, Anal Biochem (1970) 34:595). If the ninhydrin test is positive this step is repeated. The procedure is much simpler when active esters are used. The active ester (3 to 6 molar excess with respect to the resin) is directly dissolved in DMF containing a molar amount of 1--hydroxy-benzotriazol equivalent to that of the ester, and added to the reaction mixture.

If the ninhydrin test is negative the Fmoc protecting group is removed with 20% piperidine in DMF. The cycle is repeated with the rest of amino acids in such a way that the polypeptide chain grows from the C-terminal to the N-terminal amino acid.

### 4. The peptide liberation step:

The C-terminal amino acid is liberated from the resin under conditions in which the peptide is stable. The reagent employed is 95% trifluoroacetic acid, containing phenol, and ethanedithiol in proportions depending on the amino acids present in the sample. Protecting groups of side chains of the amino acid are also released with this reagent.

### 5. Purification of the peptide:

The peptide may be purified by HPLC using a reverse phase C18 column equilibrated with 0.1% (w/v) trifluoroacetic acid in water, the starting solvent. A linear gradient was established with the second solvent (0.1% (w/v) trifluoroacetic containing acetonitrile), until 70% of the latter was reached.

### Structural features of the new peptides object of the invention

The synthesized peptides have been tested with respect to the cytotoxicity they induce on lymphomononuclear cells obtained from peripheral blood of healthy donors. The incubation is carried out in the presence of different concentrations. The most active peptides are those with a proline between aa₅ and aa₇, together with aa₂ and aa₁₁ adopting the values indicated above.

The incubation of the active peptides with lymphomononuclear cells obtained from peripheral blood increases their cytotoxic activity towards K562 and Daudi cells used as tumour target cells. Control experiments using recognized immunomodulators such as human recombinant Interleukin-2 showed a similar activity in the killing of target cells K562 and Daudi cells, as that obtained with the peptides object of this invention. Proliferation of the lymphomononuclear cells is induced together with an increase in the number and proportion of CD14+, CD56+ and CD11b+ cells. These results indicate that these peptides can be used in adoptive immunotherapy for cancer, and also as reagents to evaluate the cellular responsiveness of the immune system.

Because of their small size, if compared to proteins and other complex molecules, these peptides are presumably less toxic and immunogenic, and consequently suitable as therapeutic agents in clinical disorders accompanied by alterations of the immune system, such as cancer, viral infections and immunodeficiencies. They could also be used in situations of high risk in old patients and children.

The invention is illustrated with some examples.

### EXAMPLES

### Example 1

Synthesis of peptide 
SEQ ID NO: 1
Asn-Val-Leu-Gly-Ala-Pro-Lys-Lys-Leu-Asn-Glu-Ser-Gln-Ala-Val
1               5                   10                  15
 This pentadecapeptide corresponds to the general formula indicated above when
R₁ = H, R₂ = OH, aa₂ = Val, aa₁₁ = Glu
and the rest of the amino acid residues are as specified in the example.

### 1. Preparation of the resin:

Two hundred mg of sarcosine polyamide gel resin (of 0.3 mEq/g) are treated with 7 ml of diethylenediamine with continuous stirring in a reactor for 10 hours, and washed 10 times with enough dimethylformamide. The resin is then let to react with a linkage agent. For that purpose 125.4 mg of the pentafluorophenyl ester of the 4-hydroxymethylphenoxyacetic acid (HMPA) and 48.64 mg of hydroxybenzotiazol (HOBT), both dissolved in DMF, are added and allowed to react for 90 minutes. The resin is then washed 10 times with DMF and the Kaiser ninhydrine test is performed to ascertain the absence of free amino groups.

### 2. Attachment to the first amino acid to the linkage arm of the resin:

The symmetric anhydride of valine with its amino group protected by the fluorene-methyloxycarbonyl group (Fmoc) is prepared. For that purpose 244.4 mg of the amino acid are allowed to react with 74.28 mg of dicyclohexylcarbodiimide (DCC), both dissolved in dichloromethane. The symmetric anhydride dissolved in DMF is allowed to react with the resin for 80 to 100 minutes using 4.4 mg of dimethylaminopyridine as catalytic agent. The resin is then washed with abundant dimethylformamide (DMF), and the Fmoc group is removed with 20% piperidine in DMF. The amino group is now ready to react with the carboxyl group of a new amino acid.

### 3. Incorporation of the 14 remaining amino acids.

To the resin 154.65 mg of the pentafluorophenyl ester of alanine dissolved in DMF together with 43.8 mg of 1-hydroxy-benzotriazol (HOBT) are added.

The rest of the amino acids are incorporated sequentially using their symmetric anhydrides or the active esters to achieve the reaction.

The symmetric anhydrides are prepared from the corresponding amino acids activating them with dicyclohexylcarbodiimide in dichloromethane. The coupling reaction is allowed to proceed for 90 minutes. If the ninhydrin test is negative the Fmoc protecting group is removed with 20% piperidine in DMF. The cycle is repeated with the rest of the amino acids in such a way that the polypeptide chain grows from the C-terminal to the N-terminal amino acid. For that purpose the pentafluorophenylesters of glutamine (173.16 mg), serine (171.21 mg), glutamic acid (691.66 mg), asparagine (168.62 mg), leucine (168.33 mg), lysine (205.63 mg), lysine (205.63 mg), proline (163.44 mg), alanine (154.65 mg), glycine (150.12 mg), leucine (168.33 mg), valine (163.33 mg) and asparagine (168.62 mg) were used.

### 4. The peptide liberation step.

After eliminating the last Fmoc group of the last amino acid incorporated, the bond joining the valine to the resin is cleaved as well as the protecting groups of the side chains of the amino acids in the sequence. This is achieved by adding 9 ml of the cleavage mixture (95% trifluoroacetic acid, 2.5% phenol, 2.5% ethanedithiol) and letting it to react overnight. The peptide is precipitated by the addition of ether, and washed several times with this solvent. The precipitate is recovered after complete evaporation of the solvent.

### Example 2

In the same way as described for Example 1 the following peptide is synthesized:

SEQ ID NO: 2
Ser-Leu-Lys-Asp-Asp-Pro-Ser-Val-Ser-Lys-Glu-Ile-Leu-Ala-Val
1               5                   10                  15

This pentadecapeptide corresponds to the general formula when

R₁ = H, R₂ = OH, aa₂ = Leu, aa₁₁ = Glu

The rest of the amino acids are as specified in this example.

### Example 3

In the same way as described for Example 1 the following peptide is synthesized:

SEQ ID NO: 3
Glu-Ile-Leu-Asn-Met-Pro-Asn-Leu-Asn-Glu-Glu-Gln-Arg-Asn-Val
1 5 10 15

This pentadecapeptide corresponds to the general formula when

R₁ = H, R₂ = OH, aa₂ = Ile, aa₁₁ = Glu

The rest of the amino acids are as specified in this example.

### Example 4

SEQ ID NO: 4
Asn-Gly-Phe-Ile-Gln-Ser-Leu-Lys-Asp-Asp-Pro-Ser-Val-Ser-Lys-
1 5 10 15
-Glu-Ile-Leu-Ala-Glu-Ala-Lys-Val
20

This peptide consisting of 23 amino acids residues corresponds to the general formula when

R₁ = Asn-Gly-Phe-Ile-Gln, R₂ = Ala-Lys-Val, aa₂ = Leu, aa₁₁ = Glu.

The rest of the amino acids are as specified in this example.

### STUDY OF THE BIOLOGICAL PROPERTIES OF THE PEPTIDES OBJECT OF THE INVENTION

Generation of cells with cytotoxic activity towards NK sensitive and NK resistant target cells.

### 1. Leukapheresis

Peripheral blood mononuclear cells from healthy donors were obtained by leukapheresis, processing 5 to 10 liters of whole blood, using a Fenwall CS-3000 continuous-flow cell separator (Fenwall Laboratories, Deerfield, Illinois, USA). Vascular access was obtained with a double lumen central venous catheter.

### 2. Activation of mononuclear cells in vitro

Separated lymphomononuclear cells are washed, counted and resuspended at a concentration of 2x10⁶ per ml in RPMI-1640 medium containing 10 units of penicillin per ml, 200 µg of gentamycin per ml, 2mM of glutamine per ml and 2,5% of autologous serum in a PL-732 culture bag (Fenwall Laboratories, Deerfield, Illinois, USA).

The incubation medium contained 20 micrograms of the peptide being studied.
Peptide 1 (Peptide of Example 1).
Peptide 2 (Peptide of Example 2).
Peptide 3 (Peptide of Example 3).
Peptide 4 (Peptide of Example 4).
Control Peptide (Ala-Asp-Ala-Gln-Gln-Asn-Lys-Phe-Asn-Lys-Asp-Gln-Gln-Ser-Val. In this pentadecapeptide Proline is absent. This control peptide has in common with the peptides object the invention only aa₁₁=Asp).

The cells are cultured for 4 days in a moist-air incubator at 37^{º}C, 5% CO₂ and 21% O₂, One ml sample is drawn daily for microbiological studies.

Changes in the cytotoxic ability of lymphomononuclear cells before and after peptide activation are measured with the ⁵¹Cr release assay, using K562 and Daudi target cells (Table I). Phenotypic changes of lymphomononuclear cells before and after peptide activation are also assessed with fluorescein labeled mononuclear antibodies and automated flow cytometry (Table II). It is well known that lymphomononuclear cells are very heterogeneous, but the presence of surface markers (CD) allows the description of their phenotypes (Weissman IL, Fathman CG, J Exp Med (1973) 137:504; Small M, Herzenberg CA, Weissman IL, Cell Immunol (1975) 15:109).

**Table I.**

| Peptide-induced cytolytic activity against K562 and Daudi target cells. Lymphomononuclear cells have been obtained from three different healthy donors. (Mean ± Standard error). | | |
|---|---|---|
| | CYTOTOXIC ACTIVITY (%) | |
| PEPTIDE | DAUDI | K562 |
| None | 9 ± 3 | 19 ± 5 |
| Peptide 1 | 68 ± 6 | 63 ± 8 |
| Peptide 2 | 62 ± 8 | 62 ± 7 |
| Peptide 3 | 58 ± 9 | 59 ± 9 |
| Peptide 4 | 56 ± 6 | 58 ± 7 |
| Control Peptide | 10 ± 4 | 12 ± 8 |

**Table II.**

| Phenotypes of lymphomononuclear cells after peptide activation. Lymphomononuclear cells have been obtained from three different healthy donors. (Mean ± Standard error). | | | | | | |
|---|---|---|---|---|---|---|
| | CELLS EXPRESSING SURFACE MARKERS (%) | | | | | |
| PEPTIDE | CD3 | CD8 | CD11b | CD14 | CD16 | CD56 |
| None | 59 ± 3 | 17 ± 2 | 14 ± 4 | 9 ± 1 | 11 ± 5 | 6 ± 2 |
| Peptide 1 | 60 ± 1 | 17 ± 1 | 23 ± 5 | 17 ± 4 | 22 ± 7 | 18 ± 6 |
| Peptide 2 | 58 ± 2 | 18 ± 2 | 24 ± 3 | 18 ± 3 | 22 ± 6 | 17 ± 5 |
| Peptide 3 | 59 ± 3 | 17 ± 2 | 23 ± 4 | 18 ± 3 | 21 ± 5 | 18 ± 5 |
| Peptide 4 | 58 ± 3 | 18 ± 2 | 21 ± 3 | 17 ± 4 | 21 ± 6 | 17 ± 5 |
| Control peptide | 60 ± 2 | 17 ± 2 | 13 ± 4 | 9 ± 2 | 12 ± 4 | 6 ± 2 |

### SEQUENCE LISTING

SEQ ID NO: 1
   SEQUENCE TYPE: Amino acids
      SEQUENCE LENGTH: 15 amino acids 
Asn Val Leu Gly Ala Pro Lys Lys Leu Asn Glu Ser Gln Ala Val
1               5                   10                  15
   SEQ ID NO: 2
      SEQUENCE TYPE: Amino acids
      SEQUENCE LENGTH: 15 amino acids 
Ser Leu Lys Asp Asp Pro Ser Val Ser Lys Glu Ile Leu Ala Val
1               5                   10                  15
   SEQ ID NO: 3
      SEQUENCE TYPE: Amino acids
      SEQUENCE LENGTH: 15 amino acids 
Glu Ile Leu Asn Met Pro Asn Leu Asn Glu Glu Gln Arg Asn Val
1 . 5 10 15
   SEQ ID NO: 4
      SEQUENCE TYPE: Amino acids
      SEQUENCE LENGTH: 23 amino acids

Asn Gly Phe Ile Gln Ser Leu Lys Asp Asp Pro Ser Val Ser Lys
1               5                   10                  15
Glu Ile Leu Ala Glu Ala Lys Val
                20

## Claims

1. New peptides active as immunomodulators with the general formula
R₁-aa₁-aa₂-aa₃-aa₄-aa₅-Pro-aa₇-aa₈-aa₉-aa₁₀-aa₁₁-aa₁₂-aa₁₃-aa₁₄-aa₁₅-R₂
in which
R₁ represents H, R₂ represents OH, or else R₁ and R₂ represent each an amino acid residue or peptide chains, equal or different, containing from 2 to 10 amino acid residues, in such a way that when R₁=H and R₂=OH the formula represents a pentadecapeptide in which the N-terminal and the C-terminal amino acids are respectively aa₁ and aa₁₅;
aa₂ is a Val, Leu, Ile, Ala, Lys o Gly residue;
aa₁₁ is a Glu or Asp residue;
and the rest of the amino acid residues aaₙ different from
aa₂ and aa₁₁ may be any of the 20 natural amino acids with L configuration.

2. Synthesis of peptides according to claim 1 following the procedure of solid phase for the chemical synthesis of peptides.

3. Use of the peptides defined in claim 1 for the preparation of an agent for the treatment of cancer.

4. Use of the peptides defined in claim 1 for the preparation of a medicament for the treatment of diseases due to, or accompanied by immunodepression.

5. Use of peptides active as immunomodulators according to claims 1 and 2 as laboratory reagents for the study of cellular immunity in healthy subjects and patients, and as a diagnostic tool of cancer, infections and diseases accompanied by dysfunctions of the immune system.

6. Use of the peptides defined in claim 1 for the preparation of an agent aimed at the prophylaxis of immune dysfunctions in healthy subjects and in high risk situations of old patients and children.

7. Use of the peptides defined in claim 1 for the preparation of a medicament for the treatment of immunodeficiency.

8. Use of the peptides defined in claim 1 for the preparation of a medicament for the treatment of autoimmune and degenerative diseases.

9. Use of the peptides defined in claim 1 for the preparation of an agent aimed at the obtention of activated lymphomononuclear cells to be applied for the diagnosis and for the treatment of conditions accompanied by, or due to immunologic dysfunctions.

10. Use of the peptides defined in claim 1 for the preparation of an agent for use in adoptive immunotherapy.

## Patentansprüche

1. Neue als Immunomodulatoren aktive Peptide der allgemeinen Formel
R₁-aa₁-aa₂-aa₃-aa₄-aa₅-Pro-aa₇-aa₈-aa₉-aa₁₀-aa₁₁-aa₁₂-aa₁₃-aa₁₄-aa₁₅-R₂
in der
R₁ H, R₂ OH oder R₁ und R₂ jeweils einen Aminsäurerest oder gleiche oder verschiedene Peptidketten darstellen, die 2-10 Aminsäurereste enthalten, dergestalt, daß, wenn R₁=H und R₂=OH ist, die Formel ein Pentadekapeptid darstellt, in dem die N- und C-endständigen Aminsäuren jeweils aa₁ und aa₁₅ sind;
aa₂ ist ein Val-, Leu-, Ile-, Ala-, Lys- oder Gly-Rest;
aa₁₁ ist ein Glu- oder Asp-Rest;
und die übrigen Aminsäurereste aaₙ, die sich von aa₂ und aa₁₁ unterscheiden, können jede der 20 natürlichen Aminsäuren mit L-Konfiguration sein.

2. Synthese von Peptiden nach Anspruch 1 gemäß dem Verfahren der festen Phase für die chemische Peptidsynthese.

3. Verwendung der in Anspruch 1 definierten Peptide zur Herstellung eines Mittels zur Krebsbehandlung.

4. Verwendung der in Anspruch 1 definierten Peptide zur Herstellung eines Medikaments zur Behandlung von Krankheiten als Folge einer Immunodepression oder solchen, die damit einhergehen.

5. Verwendung von als Immunomodulatoren aktiven Peptiden nach den Ansprüchen 1 und 2 als Laborreagenzien für das Studium der Zellimmunität bei Gesunden und Kranken und als Diagnosemittel für Krebs, Infektionen und Krankheiten, die von Immunsystemdisfunktionen begleitet werden.

6. Verwendung der in Anspruch 1 definierten Peptide zur Herstellung eines Mittels, das für die Prophylaxe von Immundisfunktionen bei Gesunden und in sehr riskanten Situationen bei alten Kranken und Kindern bestimmt ist.

7. Verwendung der in Anspruch 1 definierten Peptide zur Herstellung eines Medikaments für die Behandlung der Immundefizienz.

8. Verwendung der in Anspruch 1 definierten Peptide zur Herstellung eines Medikaments zur Behandlung von autoimmunen und degenerativen Krankheiten.

9. Verwendung der in Anspruch 1 definierten Peptide zur Herstellung eines Mittels, das zum Erhalt von aktivierten Lymphomononuklearzellen zur Anwendung bei der Diagnose und Behandlung von Zuständen bestimmt ist, die von immunologischen Disfunktionen begleitet oder davon verursacht werden.

10. Verwendung der in Anspruch 1 definierten Peptide zur Herstellung eines Mittels zur Anwendung bei der adoptiven Immuntherapie.

## Revendications

1. Nouveaux peptides agissant comme immunomodulateurs avec la formule générale
R₁-aa₁-aa₂-aa₃-aa₄-aa₅-Pro-aa₇-aa₈-aa₉-aa₁₀-aa₁₁-aa₁₂-aa₁₃-aa₁₄-aa₁₅-R₂
dans laquelle
R₁ représente H, R₂ représente OH, ou R₁ et R₂ représentent chacun un résidu aminoacide ou des chaînes peptidiques, identiques ou différentes, qui contiennent de 2 à 10 résidus aminoacides, de telle sorte que lorsque R₁=H et R₂=OH la formule représente un pentadécapeptide dans lequel les aminoacides N-terminal et C-terminal sont respectivement aa₁ et aa₁₅;
aa₂ est un résidu Val, Leu, Ile, Ala, Lys ou Gly;
aa₁₁ est un résidu Glu ou Asp;
et le reste des résidus aminoacides aaₙ autres que aa₂ et aa₁₁ peut être aussi n'importe lequel des 20 aminoacides naturels avec la configuration L.

2. Synthèse de peptides conformément à la revendication 1 suivant le procédé de phase solide pour la synthèse chimique des peptides.

3. Utilisation des peptides définis dans la revendication 1 pour préparer un agent pour le traitement du cancer.

4. Utilisation des peptides définis dans la revendication 1 pour préparer un médicament pour le traitement de maladies dues à ou accompagnées d'immunodépression.

5. Utilisation de peptides agissant comme immunomodulateurs conformément aux revendications 1 et 2 comme réactifs de laboratoire pour l'étude d'immunité cellulaire sur des sujets sains et patients, et comme un instrument de diagnostic du cancer, des infections et des maladies accompagnées de dysfonctionnements du système d'immunité.

6. Utilisation des peptides définis dans la revendication 1 pour préparer un agent destiné à la prophylaxie de dysfonctionnements d'immunité sur des sujets sains et dans des situations de risque élevé chez des patients âgés et des enfants.

7. Utilisation des peptides définis dans la revendication 1 pour préparer un médicament pour le traitement de l'immunodéficience.

8. Utilisation des peptides définis dans la revendication 1 pour préparer un médicament pour le traitement de maladies d'autoimmunité et dégénératives.

9. Utilisation des peptides définis dans la revendication 1 pour préparer un agent destiné à obtenir des cellules lymphomononucléaires activées pour leur application dans le diagnostic et le traitement de conditions accompagnées de ou dues à des dysfonctionnements immunologiques.

10. Utilisation des peptides définis dans la revendication 1 pour préparer un agent à employer dans l'immunothérapie adoptive.
